# EUROPÄISCHE PATENTSCHRIFT

(11) **EP 0 394 644 B1**
(45) Veröffentlichungstag und Bekanntmachung des Hinweises auf die Patenterteilung: **05.10.1994**
(21) Anmeldenummer: 90104397.6
(22) Anmeldetag: 08.03.1990
(51) Int. Cl.: C07C 255/35, C07C 255/57, C07C 255/41, C07C 25/13, C07C 57/58, C07C 69/65

(54) **Halogenbenzolderivate**
Halobenzene derivatives
Dérivés de halogenobenzènes

(30) Priorität: 21.03.1989 DE 3909213
(43) Veröffentlichungstag der Anmeldung: 31.10.1990
(73) Patentinhaber: BAYER AG, 51368 Leverkusen (DE)
(72) Erfinder: Marhold, Albrecht, Dr., D-5090 Leverkusen 1 (DE); Fischer, Reiner, Dr., D-4019 Monheim 2 (DE)

(56) Entgegenhaltungen:
- GB-A- 901 880
- US-A- 3 732 278
- CHEMICAL ABSTRACTS, Band 88, Nr. 15, 10. April 1978, S. 495, Zusammenfassung Nr. 104464n, Columbus, Ohio, USA; V. M. VLASOV et al: "Polyfluoroaryl-containing enolate anions. Fluorine-19 NMR spectra and relative stability"
- CHEMICAL ABSTRACTS, Band 88, 2.-16. Januar 1978, S. 519, Zusammenfassung Nr. 6108e, Columbus, Ohio, USA; V. M. VLASOV et al: "Mesomeric carbanions of polyfluoroarylmethanes. Fluorine-19 NMR spectra and the scale of relative stability"
- CHEMICAL ABSTRACTS, Band 83, 7.-14. Juli 1975, S. 742, Zusammenfassung Nr. 8924p, Columbus, Ohio, USA; V. M. VLASOV et al: "Fluorine-19 NMR spectra and relative thermodynamic stability of mesomeric carbanions obtained from polyfluorophenylmalonic acid derivatives"

## Beschreibung

Die vorliegende Erfindung betrifft Halogenbenzolderivate, die weiter unten durch Formel (I) näher gekennzeichnet werden und ein Verfahren zu deren Herstellung.

Es ist bekannt, daß man Fluorbenzolderivate, insbesondere Trifluormethyl-substituierte Phenylessigsäuren herstellen kann, indem man Trifluormethylbenzylhalogenide mit Cyaniden umsetzt und die dabei sich bildenden Benzylcyanide anschließend verseift (siehe Organicum, 8. Auflage, Berlin 1968, Seite 412). Nachteilig bei diesem Verfahren ist die schwere Zugänglichkeit von 4-Trifluormethylbenzylhalogeniden, insbesondere wenn der aromatische Kern ebenfalls durch Fluor substituiert sein solle. Weiterhin kann man durch Meerwein-Arylierung von Vinylidenchlorid mit entsprechenden Aryldiazoniumchloriden und nachfolgender Verseifung der so erhältlichen Trichlorethylbenzotrifluoride Trifluormethyl-substituierte Phenylessigsäuren erhalten (siehe US-PS 4 426 536). Bei diesem Verfahren sind die benötigten 4-Trifluormethylaniline zwar relativ gut zugänglich, jedoch bedeutet deren Synthese eine zusätzliche Stufe verbunden mit entsprechenden Ausbeuteverlusten. Schließlich kann man Trifluormethyl-substituierte Phenylessigsäuren auch ausgehend von Trifluormethylbenzylhalogeniden über die Stufen Benzoylcyanid, Phenylglyoxylsäure und deren katalytische Hydrierung erhalten. Hierbei werden ebenfalls schwer zugängliche Trifluormethylbenzoylhalogenide benötigt und nur geringe Ausbeuten erzielt.

Es wurden nun Halogenbenzolderivate der Formel (I) gefunden 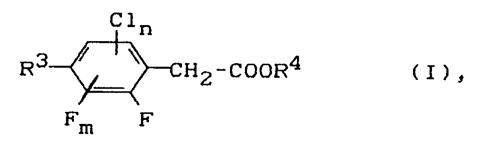
in der
- R³: für CF₃ oder Cl,
- R⁴: für H oder C₁- bis C₄-Alkyl und
- m und n: unabhängig voneinander für 0, 1 oder 2 stehen,
wobei m + n mindestens 1 und höchstens 3 beträgt.

Wichtige Einzelverbindungen der Formel (I) sind Phenylessigsäureester mit R⁴ = CH₃ oder C₂H₅ und
Phenylessigsäuren mit R⁴ = H,
bei denen jeweils R³ für CF₃ steht und jeweils folgende weitere Substituenten vorhanden sind:
2,3-Difluor, 2,5-Difluor, 2,6-Difluor, 2-Fluor-5-chlor, 2-Fluor-6-chlor, 2,3,5-Trifluor und 2,3,5-Trifluor-6-chlor.

Die vorliegende Erfindung betrifft auch ein Verfahren zur Herstellung von Halogenbenzolderivaten der Formel (I), das dadurch gekennzeichnet ist, daß man ein Polyhalogenbenzol der Formel (II) 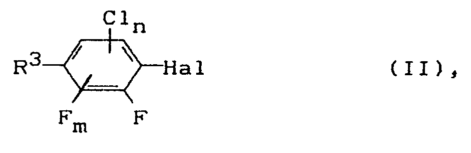
in der
- R³, m und n: die bei Formel (I) angegebene Bedeutung haben,
und
- Hal: für ein (weiteres) Fluor- oder Chloratom steht,
in Gegenwart einer Base und eines Lösungsmittels mit einem Ester der Formel (III) umsetzt

R⁵-CH₂-COOR⁴ (III),

in der
- R⁴: die bei Formel (I) angegebene Bedeutung hat und
- R⁵: für CN oder COOR⁴ stehen,
so eine Verbindung der Formel (IV) erhält 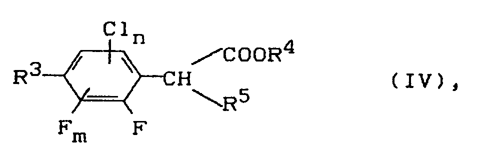
in der
R³, R⁴, R⁵, m und n die oben angegebene Bedeutung haben und diese sauer hydrolysiert.

Polyhalogenbenzole der Formel (II) sind bekannt und beispielsweise gemäß DE-OS 3 725 659 zugänglich oder auf analoge Weise dazu. Ester der Formel (III) sind ebenfalls bekannt. Es handelt sich dabei um Cyanessigsäureester (R⁵ = CN) oder Malonsäureester (R⁵ = COOR⁴). Bevorzugt sind die Methyl- und Ethylester, insbesondere Cyanessigsäuremethylester und Cyanessigsäureethylester.

Die Umsetzung eines Polyhalogenbenzols der Formel (II) mit einem Ester der Formel (III) erfolgt in Gegenwart einer Base und eines Lösungsmittels. Geeignete Basen sind beispielsweise Alkalicarbonate, Alkalihydroxide, Ammoniumcarbonat, Alkalihydride, Erdalkalihydride, Kaliumfluorid, Cäsiumfluorid, organische Stickstoffbasen, Alkalialkoholate und Erdalkalialkoholate.

Bevorzugt sind Kaliumcarbonat und Natriumhydrid. Beispielsweise kann man pro Mol Polyhalogenbenzol der Formel (II) 0,5 bis 2 Äquivalente Base einsetzen. Vorzugsweise beträgt diese Menge 1 bis 1,5 Äquivalente.

Geeignete Lösungsmittel sind beispielsweise Ether und Polyether wie Glyme, Dioxan und Diethylenglykoldimethylether, Amide wie Dimethylformamid, Dimethylacetamid, N-Methylpyrrolidon und N-Methylcaprolactam, sowie Sulfone wie Tetramethylensulfon.

Bevorzugt ist Dimethylformamid, N-Methylpyrrolidon und Dioxan. Geeignete Temperaturen für diese Umsetzung sind beispielsweise solche von 20 bis 150°C. Bevorzugt arbeitet man bei 80 bis 130°C. Vorzugsweise führt man diese Umsetzung unter striktem Ausschluß von Wasser durch.

Die auf diese Weise erhältlichen Verbindungen der Formel (IV) können aus dem Reaktionsgemisch isoliert werden, beispielsweise indem man zunächst das Lösungsmittel abzieht, den verbleibenden Rückstand mit Wasser versetzt, durch Zugabe von Säure einen pH-Wert im Bereich von 3 bis 7,5 einstellt und dann entweder den ausfallenden Feststoff absaugt und trocknet oder die Verbindung der Formel (IV) mit einem organischen Lösungsmittel extrahiert und durch Destillation gewinnt.

Man erhält so Verbindungen der Formel (IV), bei denen ein Fluor- oder Chloratom aus dem eingesetzten Polyhalogenbenzol der
Formel (II) durch eine 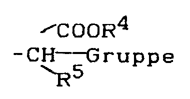
ersetzt ist und die sonst im allgemeinen das gleiche Substitutionsmuster aufweisen wie das eingesetzt Polyhalogenbenzol der Formel (II). Anschließend an die Umsetzung des Polyhalogenbenzols der Formel (II) mit Estern der Formel (III) ist noch eine saure Hydrolyse durchzuführen.

Verbindungen der Formel (I), bei denen R⁴ für H steht, erhält man aus Verbindungen der Formel (IV), indem man diese beispielsweise mit einem Gemisch aus konzentrierter Mineralsäure und wäßriger Essigsäure bei 80 bis 120°C verseift. Bevorzugt wendet man dafür Schwefelsäure in einer Konzentration über 70 Gew.-% und wäßrige Essigsäure und 100 bis 120°C an.

Verbindungen der Formel (I), bei denen R⁴ für C₁- bis C₄-Alkyl steht, erhält man, indem man Verbindungen der Formel (IV) mit Mineralsäure bei 60 bis 120°C verseift. Bevorzugt wendet man hierfür 70 bis 90 gew.-%ige Schwefelsäure in Abwesenheit von Essigsäure und 80 bis 120°C an.

Es kann in bestimmten Fällen vorteilhaft sein, eine gewünschte Verbindung der Formel (I) in einer Abwandlung einer der oben beschriebenen Arbeitsweisen herzustellen, beispielsweise indem man ein gewünschtes Substitutionsmuster nicht durch den Einsatz eines entsprechenden Polyhalogenbenzols der Formel (II) und eines entsprechenden Esters der Formel (III) realisiert, sondern nach der Umsetzung des Polyhalogenbenzols der Formel (II) mit einem Ester der Formel (III) eine zusätzliche Umderivatisierung vornimmt.

Ein Beispiel hierfür ist der Einsatz von chlorhaltigen Polyhalogenbenzolen der Formel (II) (z. B. mit n = 1 oder 2) auch zur Herstellung chlorfreier oder chlorärmerer Halogenbenzolderivate der Formel (I). Chlorhaltige Polyhalogenbenzole der Formel (II) lassen sich häufig besser mit Estern der Formel (III) umsetzen als chlorfreie Polyhalogenbenzole. Nach dieser Umsetzung kann man dann unerwünschtes Chlor durch eine, gegebenenfalls partielle, Dechlorierung entfernen. Dies kann man beispielsweise erreichen, indem man ein Lösungsmittel und einen Hydrierkatalysator zufügt und bei Raumtemperatur oder erhöhter Temperatur Wasserstoff aufdrückt. Nach Entfernung des Katalysators und des Lösungsmittels kann man das dechlorierte Produkt durch Destillation oder Extraktion gewinnen.

Das erfindungsgemäße Verfahren zur Herstellung von Halogenbenzolderivaten der Formel (I), bei dem eine wesentliche Maßnahme die Umsetzung eines Polyhalogenbenzols der Formel (II) mit einem Ester der Formel (III) ist, geht von gut zugänglichen Ausgangsprodukten aus, ist auf einfache Weise durchführbar und liefert die gewünschten Halogenbenzolderivate der Formel (I) in guten Ausbeuten. Dabei ist besonders überraschend, daß die Esterkondensation und die sich anschließende Verseifung sehr selektiv verlaufen. Durch die gegebenen vielfältigen Variationsmöglichkeiten, z.B. hinsichtlich der Polyhalogenbenzole der Formel (II) als Ausgangsprodukte und nach der Esterkondensation vornehmbarer Umwandlungsreaktionen wird eine breite Zugänglichkeit zu den Halogenbenzolderivaten der Formel (I) zur Verfügung gestellt.

Die Halogenbenzolderivate der Formel (I) sind wertvolle Zwischenprodukte zur Herstellung von Pflanzenschutzwirkstoffen. Beispielsweise kann man 3-Aryl-pyrrolidin-2,4-dion-Derivate der Formel (V) 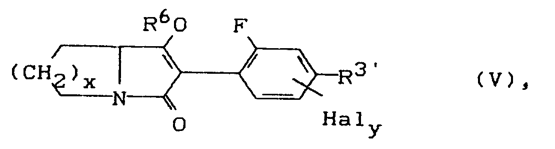
in der
- x: für eine ganze Zahl von 1 bis 4,
- R^{3'}: für CF₃ oder Cl,
- Hal: für F oder Cl
- y: für 0 oder eine ganze Zahl von 1 bis 3 und
- R⁶: für Alkyl steht,
herstellen,
indem man zunächst auf an sich bekannte Weise, z. B. durch Umsetzung mit Thionylchlorid, eine Verbindung der Formel (I) in eine solche der Formel (VI) überführt 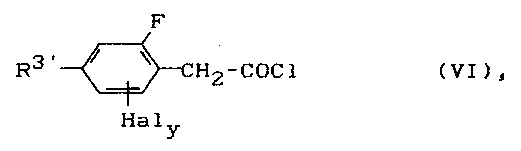
in der
R^{3'}, Hal und y die bei Formel (V) angegebene Bedeutung haben und dann damit einen Aminosäureester der Formel (VII) 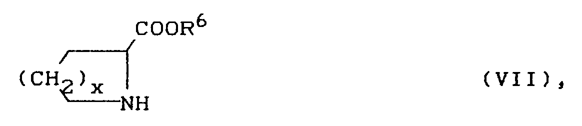
in der
x und R⁶ die bei Formel (V) angegebene Bedeutung haben,
acyliert, so eine Verbindung der Formel (VIII) 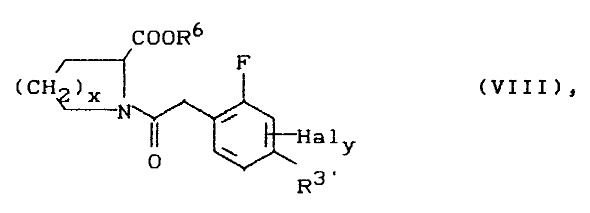
in der die verwendeten Symbole die weiter oben angegebene Bedeutung haben,
erhält, und diese in Gegenwart eines Verdünnungsmittels und einer Base intramolekular kondensiert. Die 3-Aryl-pyrrolidin-2,4-dion-Derivate der Formel (V) können als Herbizide, Fungizide, Insektizide und Akarizide verwendet werden. Verbindungen der Formel (V), deren Herstellung und deren Verwendung im Pflanzenschutz sind Gegenstand der eigenen älteren europäischen Patentanmeldung Nr. 89114789.4, inzwischen EP-B 355 599.

### Beispiele

Die Beispiele 1 bis 3 betreffen die Herstellung von Ausgangsmaterialien.

### Beispiele 1 bis 3

In 300 ml trockenem Dimethylformamid wurden 55 g gepulvertes Kaliumcarbonat vorgelegt und 0,314 Mol fluoriertes oder fluoriertes und chloriertes Benzotrifluorid zusammen mit 0,4 Mol Cyanessigsäuremethylester zugegeben. Anschließend wurde unter Feuchtigkeitsausschluß für 10 Stunden auf 120°C erhitzt. Nach dem Abdestillieren des Dimethylformamids bei 15 mbar wurde der Rückstand mit 250 ml Wasser verrührt. Dann wurde Salzsäure bis zur schwach sauren Reaktion zugetropft und anschließend das Festprodukt abgesaugt und getrocknet. Einzelheiten sind aus Tabelle 1 ersichtlich.

**Tabelle 1**

| Beispiel Nr. | Einsatzmaterial 4-Fluoro-benzotrifluorid | Reaktionsprodukt α-Cyano-α-(4-trifluoromethyl-phenyl)-essigsäure-methylester | Schmelzpunkt (°C) | Ausbeute (% d.Th.) |
|---|---|---|---|---|
| 1 | 3-chlor-5-fluor | 2-fluor-6-chlor | 64-65 | 86 |
| 2 | 3-fluor-6-chlor | 2-fluor-5-chlor | 51-52 | 93,5 |
| 3 | 2,6-difluor | 3,5-difluor | 83-84 | 68 |

Die Beispiele 4 bis 6 betreffen die Herstellung von Verbindungen der Formel (I)

### Beispiele 4 bis 6

Zu einer Mischung aus 85 ml Wasser, 85 ml Essigsäure und 125 ml Schwefelsäure (96 %ig) wurden 0,15 Mol fluorierter oder fluorierter und chlorierter Arylcyanessigsäureester gegeben und anschließend für 8 Stunden auf 120°C erhitzt. Nach dem Abkühlen wurde das Reaktionsgemisch in 200 ml Wasser eingerührt und die ausgefallene Phenylessigsäure abgesaugt. Einzelheiten sind aus Tabelle 2 ersichtlich.

**Tabelle 2**

| Beispiel Nr. | Einsatzmaterial Produkt aus Beispiel | Reaktionsprodukt 4-Trifluoromethylphenyl-essigsäure | Schmelzpunkt (°C) | Ausbeute (% d.Th.) |
|---|---|---|---|---|
| 4 | 1 | 2-fluor-6-chlor | 119 | 72 |
| 5 | 2 | 2-fluor-5-chlor | 63-65 | 67 |
| 6 | 3 | 2,5-difluor | 74-75 | 67 |

## Patentansprüche

1. Halogenbenzolderivate der Formel (I) 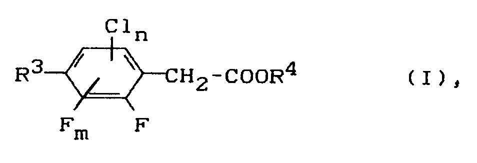 in der
R³ für CF₃ oder Cl,
R⁴ für H oder C₁- bis C₄-Alkyl und
m und n unabhängig voneinander für 0, 1 oder 2 stehen,
wobei m + n mindestens 1 und höchstens 3 beträgt.

2. Halogenbenzolderivate gemäß Anspruch 1, dadurch gekennzeichnet, daß es sich handelt um
Phenylessigsäureester mit R⁴ = CH₃ oder C₂H₅ und
Phenylessigsäuren mit R⁴ = H,
bei denen R³ jeweils für CF₃ steht und jeweils folgende weitere Substituenten vorhanden sind:
2,3-Difluor, 2,5-Difluor, 2,6-Difluor, 2-Fluor-5-chlor, 2-Fluor-6-chlor, 2,3,5-Trifluor oder 2,3,5-Trifluor-6-chlor.

3. Verfahren zur Herstellung von Halogenbenzolderivaten des Anspruchs 1, dadurch gekennzeichnet, daß man ein Polyhalogenbenzol 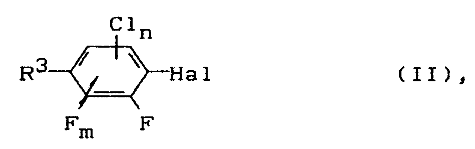 in der
R³, m und n die in Anspruch 1 angegebene Bedeutung haben,
und
Hal für ein (weiteres) Fluor- oder Chloratom steht,
in Gegenwart einer Base und eines Lösungsmittels mit einem Ester der Formel (III) umsetzt
R⁵-CH₂-COOR⁴ (III),
in der
R⁴ die in Anspruch 1 angegebene Bedeutung hat und
R⁵ für CN oder COOR⁴ stehen,
so eine Verbindung der Formel (IV) erhält 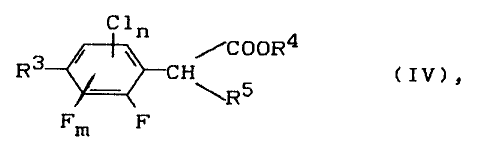 in der
R³, R⁴, R⁵, m und n die oben angegebene Bedeutung haben und diese sauer hydrolysiert.

4. Verfahren nach Anspruch 3, dadurch gekennzeichnet, daß als Base Alkalicarbonate, Alkalihydroxide, Ammoniumcarbonat, Alkalihydride, Erdalkalihydride, Kaliumfluorid, Cäsiumfluorid, organische Stickstoffbasen, Alkalialkoholate oder Erdalkalialkoholate in einer Menge von 0,5 - 2 Äquivalenten, bezogen auf 1 Mol Polyhalogenbenzol der Formel (II), eingesetzt werden.

5. Verfahren nach Ansprüchen 3 und 4, dadurch gekennzeichnet, daß als Lösungsmittel Ether, Polyether, Amide oder Sulfone eingesetzt werden.

6. Verfahren nach Ansprüchen 3 bis 5, dadurch gekennzeichnet, daß man Verbindungen der Formel (IV) mit einem Gemisch aus konzentrierter Mineralsäure und wäßriger Essigsäure bei 80 - 120°C verseift.

7. Verfahren nach Ansprüchen 3 bis 5, dadurch gekennzeichnet, daß man Verbindungen der Formel (IV) mit Mineralsäure bei 60 -120°C verseift.

8. Verfahren nach Ansprüchen 3 bis 7, dadurch gekennzeichnet, daß man nach der Umsetzung eines Polyhalogenbenzols der Formel (II) mit einem Ester der Formel (III) eine Umderivatisierung vornimmt.

## Claims

1. Halogenobenzene derivatives of the formula (I) 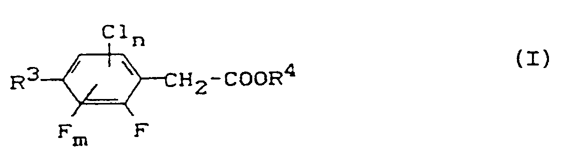 in which
R³ represents CF₃ or Cl,
R⁴ represents H or C₁- to C₄-alkyl and
m and n independently of one another represent 0, 1 or 2,
wherein m + n is at least 1 and not more than 3.

2. Halogenobenzene derivatives according to Claim 1, characterized in that they are
phenylacetic acid esters where R⁴ = CH₃ or C₂H₅ and
phenylacetic acids where R⁴ = H,
in which in each case R³ represents CF₃ and in each case the following further substituents are present:
2,3-difluoro, 2,5-difluoro, 2,6-difluoro, 2-fluoro-5-chloro, 2-fluoro-6-chloro, 2,3,5-trifluoro or 2,3,5-trifluoro-6-chloro.

3. Process for the preparation of halogenobenzene derivatives of Claim 1, characterized in that a polyhalogenobenzene 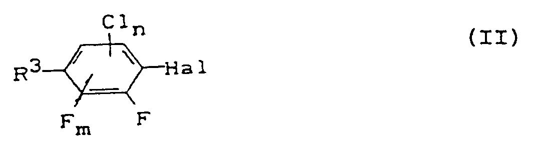 in which
R³, m and n have the meaning given in Claim 1
and
Hal represents a (further) fluorine or chlorine atom,
is reacted with an ester of the formula (III)
R⁵-CH₂-COOR⁴ (III)
in which
R⁴ has the meaning given in Claim 1 and
R⁵ represents CN or COOR⁴,
in the presence of a base and a solvent, a compound of the formula (IV) 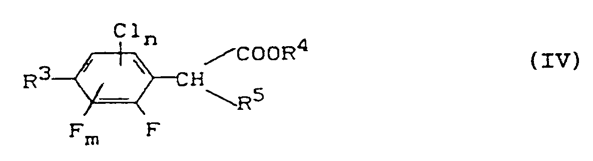 is thus obtained, in which
R³, R⁴, R⁵, m and n have the abovementioned meaning, and this compound is subjected to acid hydrolysis.

4. Process according to Claim 3, characterized in that alkali metal carbonates, alkali metal hydroxides, ammonium carbonate, alkali metal hydrides, alkaline earth metal hydrides, potassium fluoride, caesium fluoride, organic nitrogen bases, alkali metal alcoholates or alkaline earth metal alcoholates are employed as the base in an amount of 0.5 - 2 equivalents per mol of polyhalogenobenzene of the formula (II).

5. Process according to Claims 3 and 4, characterized in that ethers, polyethers, amides or sulphones are employed as the solvent.

6. Process according to Claims 3 to 5, characterized in that compounds of the formula (IV) are hydrolyzed with a mixture of concentrated mineral acid and aqueous acetic acid at 80 - 120°C.

7. Process according to Claims 3 to 5, characterized in that compounds of the formula (IV) are hydrolyzed with mineral acid at 60 - 120°C.

8. Process according to Claims 3 to 7, characterized in that after the reaction of a polyhalogenobenzene of the formula (II) with an ester of the formula (III), a trans-derivatization is carried out.

## Revendications

1. Dérivés d'halogénobenzènes de formule (I) 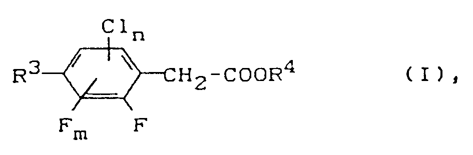 dans laquelle
R³ représente CF₃ ou Cl,
R⁴ représente H ou alkyle en C₁-C₄ et
m et n représentent indépendamment l'un de l'autre 0, 1 ou 2, la somme m + n étant d'au moins 1 et au plus 3.

2. Dérivés d'halogénobenzènes selon la revendication 1, caractérisés en ce qu'il s'agit d'esters phénylacétiques dans lesquels R⁴ = CH₃ ou C₂H₅ et d'acides phénylacétiques dans lesquels R⁴ = H, dans lesquels R³ représente chaque fois CF₃ et contenant chaque fois les autres substituants suivants :
2,3-difluoro, 2,5-difluoro, 2,6-difluoro, 2-fluoro-5-chloro, 2-fluoro-6-chloro, 2,3,5-trifluoro et 2,3,5-trifluoro-6-chloro.

3. Procédé pour la fabrication de dérivés d'halogénobenzènes selon la revendication 1, caractérisé en ce que l'on fait réagir un polyhalogénobenzène de formule (II) 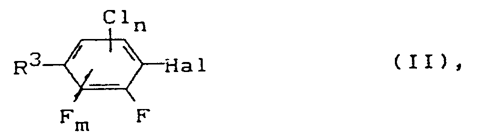 dans laquelle
R³, m et n ont la signification indiquée dans la revendication 1 et
Hal représente un (autre) atome de fluor ou de chlore,
en présence d'une base et d'un solvant avec un ester de formule (III) :
R⁵-CH₂-COOR⁴ (III),
dans laquelle
R⁴ a la signification indiquée dans la revendication 1 et R⁵ représente CN ou COOR⁴,
on obtient ainsi un composé de formule (IV) 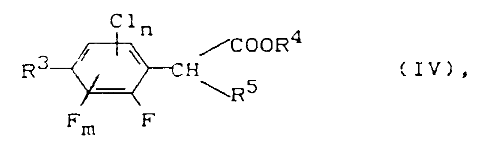 dans laquelle
R³, R⁴, R⁵, m et n ont la signfiication indiquée ci-dessus et on hydrolyse celui-ci par un acide.

4. Procédé selon la revendication 3, caractérisé en ce que l'on utilise comme bases des carbonates alcalins, des hydroxydes alcalins, le carbonate d'ammonium, des hydrures alcalins, les hydrures alcalino-terreux, le fluorure de potassium, le fluorure de césium, des bases azotées organiques, des alcoolates alcalins et des alcoolates alcalino-terreux de formule (II) en quantités de 0,5 à 2 équivalents de la base.

5. Procédé selon les revendications 3 et 4, caractérisé en ce que l'on utilise comme solvants des éthers, des polyéthers, des amides ou des sulfones.

6. Procédé selon les revendications 3 à 5, caractérisé en ce que l'on saponifie les composés de formule (IV) avec un mélange d'acide minéral concentré et d'acide acétique aqueux à 80-120°C.

7. Procédé selon les revendications 3 à 5, caractérisé en ce que l'on saponifie les composés de formule (IV) avec un acide minéral à 60-120°C.

8. Procédé selon les revendications 3 à 7, caractérisé en ce que l'on effectue un changement de dérivé après la réaction d'un polyhalogénobenzène de formule (II) avec un ester de formule (III).
